# EUROPÄISCHE PATENTSCHRIFT

(11) **EP 3 077 073 B1**
(45) Veröffentlichungstag und Bekanntmachung des Hinweises auf die Patenterteilung: **21.02.2018**
(21) Anmeldenummer: 14809324.8
(22) Anmeldetag: 01.12.2014
(51) Int. Cl.: C07C 7/00, B01D 17/04, B01D 17/00

(54) **ABTRENNUNG VON IONISCHEN FLÜSSIGKEITEN IN KOALESZIERVORRICHTUNGEN**
SEPARATION OF IONIC LIQUIDS IN COALESCING DEVICES
SÉPARATION DE LIQUIDES IONIQUES DANS DES DISPOSITIFS DE COALESCENCE

(30) Priorität: 02.12.2013 EP 13195321
(43) Veröffentlichungstag der Anmeldung: 12.10.2016
(73) Patentinhaber: BASF SE, 67056 Ludwigshafen am Rhein (DE)
(72) Erfinder: PFEIFFER, Daniel, 67435 Neustadt (DE); BITTERLICH, Stefan, 67246 Dirmstein (DE); HÜBNER, Michael, 69469 Weinheim (DE)
(86) Internationale Anmeldenummer: PCT/EP2014/076072
(87) Internationale Veröffentlichungsnummer: WO 2015/082388

(56) Entgegenhaltungen:
- DE-A1-102005 029 423
- US-A1- 2010 130 800

## Beschreibung

Die vorliegende Erfindung betrifft ein Verfahren gemäß Anspruch 1. Bei der Koalesziervorrichtung (KV) handelt es sich um einen Koaleszierfilter, gegebenenfalls können auch zwei oder mehr Koalesziervorrichtungen (KV) in Reihe oder vorzugsweise parallel geschaltet sein.

Ionische Flüssigkeiten eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan.

Im Allgemeinen sind ionische Flüssigkeiten einerseits und Kohlenwasserstoffe (bzw. organische Phasen generell) andererseits nicht oder nur sehr schwer mischbar, sie bilden zwei getrennte Phasen aus. Um die genannte Katalysewirkung nutzen zu können, muss ein intensiver Kontakt zwischen organischer Phase und der ionischer Flüssigkeit hergestellt werden. Hierzu werden die beiden Phasen häufig in Rührkesseln und intensivem Rühren unter Erhalt von Dispersionen durchmischt. In Abhängigkeit von Parametern wie Art der ionischen Flüssigkeit bzw. der organischen Phase oder dem Phasenverhältnis kann die Dispersion entweder als Dispersion einer ionischen Flüssigkeit in der organischen Phase vorliegen oder es kann sich um eine Dispersion der organischen Phase in der ionischen Flüssigkeit handeln. Unabhängig von der konkret vorliegenden Dispergierrichtung ist es bei solchen Dispersionen ein generelles Problem, die dispergierte Phase im Anschluss an die Reaktion von der zusammenhängenden Phase abzutrennen. Problematisch ist dabei insbesondere die Fallkonstellation, bei der Feinsttropfen (d < 900 µm) der ionischen Flüssigkeit aus einer Dispersion abgetrennt werden sollen, in der die ionische Flüssigkeit in der organischen Phase dispergiert ist (Feinsttropfenproblem).

Zur Auftrennung von zwei- oder mehrphasigen Gemischen, insbesondere von Dispersionen, ist die Verwendung von Koaleszierfiltern seit längerem bekannt. Beispielsweise wird in WO 2012/104769 ein Verfahren zur Reduzierung des Wassergehaltes in Pyrolysebenzin unter Verwendung eines Koaleszierfilters, der aus Metall und/oder Glasfaser hergestellt ist, offenbart. Ein Koaleszierfilter kann jedoch nicht nur zur Wasserabtrennung aus Gemischen (Dispersionen) mit einer organischen Phase (Pyrolysebenzin) verwendet werden, sondern auch zur Abtrennung von ionischen Flüssigkeiten aus Dispersionen mit einer organischen Phase.

WO 2010/062922 offenbart ein mehrstufiges Verfahren zur Trennung einer ionischen Flüssigkeit von Kohlenwasserstoffen unter Verwendung eines Koaleszierfilters. Das Koaleszierfiltermaterial muss so beschaffen sein, dass es eine stärkere Affinität für die ionische Flüssigkeit als für die Kohlenwasserstoffe hat. Als Koaleszierfiltermaterialien eignen sich gemäß WO 2010/062922 Glaskugeln, Edelstahl, Glasfasern, Polymerfasern oder organische Membrane, insbesondere Glasfasern. Im Koaleszierfilter wird eine Trennung der ionischen Flüssigkeit aus den Kohlenwasserstoffen bewirkt.

Die internationale Anmeldung PCT/EP 2013/064459 (angemeldet am 9. Juli 2013) betrifft ein Verfahren zur Abtrennung einer Phase (A), die mindestens eine ionische Flüssigkeit enthält, von einer Phase (B) in einem Koaleszierfilter, der aus Acrylphenolharz hergestellt ist. Die Phase (A) weist dabei eine höhere Viskosität auf als die Phase (B), die beispielsweise einen Kohlenwasserstoff wie Cyclohexan enthält. Bei dem Verfahren wird eine Dispersion (D1), in der die Phase (A) in der Phase (B) dispergiert ist, als Bestandteil eines Stromes (S1) in den entsprechenden Koaleszierfilter eingeleitet. Ein sinngemäßes Verfahren wird in der internationalen Anmeldung PCT/EP 2013/064432 (angemeldet am 9. Juli 2013) offenbart. In diesem Verfahren erfolgt die Abtrennung der Phase (A) von der Phase (B) unter Verwendung einer Phasentrenneinheit, die ein Gestrick enthält, anstelle der Verwendung eines Koaleszierfilters, der aus Acrylphenolharz hergestellt ist (gemäß PCT/EP 2013/064459).

In US 2010/130800 wird ein Verfahren zur Abtrennung von ionischen Flüssigkeiten von Kohlenwasserstoffen offenbart, bei dem ein Gemisch aus Kohlenwasserstoffen und ionischer Flüssigkeit in eine Koalesziervorrichtung überführt wird, wobei die ionische Flüssigkeit als kleine Tropfen in dem Kohlenwasserstoff dispergiert ist. Mindestens ein Teil der ionischen Flüssigkeitstropfen werden in der Koalesziervorrichtung gebunden, zu größeren Tropfen vereinigt und die vereinigten Tropfen der ionischen Flüssigkeit werden von dem Kohlenwasserstoff abgetrennt. DE2005029423 A1 offenbart die Benutzung eines Koaleszierfilters aus Acrylphenolharz. Nirgendwo im Stand der Technik ist jedoch beschrieben, mit welcher Anströmgeschwindigkeit die Dispersion, in der beispielsweise eine ionische Flüssigkeit (Phase (A)) in einem Kohlenwasserstoff (Phase (B)) dispergiert ist, auf die Koalesziervorrichtung, beispielsweise ein Koaleszierfilter oder ein Gestrick, trifft.

Die der vorliegenden Erfindung zugrunde liegende Aufgabe besteht in der Bereitstellung eines neuen Verfahrens zur Abtrennung einer ionischen Flüssigkeit aus einer organischen Phase, wobei die ionische Flüssigkeit in der organischen Phase dispergiert ist.

Gelöst wird die Aufgabe durch ein Verfahren zur Abtrennung einer Phase (A), die mindestens eine ionische Flüssigkeit enthält, von einer Phase (B), wobei die Phase (A) eine höhere Viskosität als die Phase (B) aufweist, umfassend die folgenden Schritte:
a) Bereitstellung eines Stroms (S1) enthaltend eine Dispersion (D1), in der die Phase (A) in der Phase (B) dispergiert ist,
b) Einleiten des Stroms (S1) in eine Koalesziervorrichtung (KV), wobei die Anströmgeschwindigkeit des Stroms (S1) 0,05 bis 150 kg/(cm² * h) bezogen auf die mittlere Querschnittsfläche der Koalesziervorrichtung (KV) beträgt, wobei die Packungsdichte der Koalesziervorrichtung (KV) 50 bis 500 kg/m³ beträgt, wobei die Koalesziervorrichtung (KV) ein Koaleszierfilter ist und das Filtermaterial des Koaleszierfilters mindestens 50 Gew.-% Acrylphenolharz enthält,
c) Abtrennen der dispergierten Phase (A) von der Phase (B) in der Koalesziervorrichtung (KV),
d) Ausleiten eines Stroms (S2) umfassend mindestens 70 Gew.-%, bevorzugt mindestens 90 Gew.-% an Phase (A) aus der Koalesziervorrichtung (KV) und
e) Ausleiten eines Stroms (S3) umfassend mindestens 70 Gew.-%, bevorzugt mindestens 90 Gew.-% an Phase (B) aus der Koalesziervorrichtung (KV).

Durch das erfindungsgemäße Verfahren kann in vorteilhafter Weise eine effektive Abtrennung von ionischen Flüssigkeiten aus Dispersionen mit organischen Phasen, insbesondere aus Dispersionen mit Kohlenwasserstoffen, erzielt werden. Erfindungsgemäß kann insbesondere das Problem der Abtrennung von in feindisperser Form und/oder in kleinen Mengen in einer Dispersion mit einer organischen Phase vorliegenden ionischen Flüssigkeit gelöst werden (Feinsttropfenproblem).

Es können durch das erfindungsgemäße Verfahren auch kleinere Mengen (< 1 Gew.-%) an ionischer Flüssigkeit aus einer Dispersion mit einer organischen Phase, insbesondere mit einer Kohlenwasserstoffphase, abgetrennt werden; dies gilt insbesondere auch für den Fall, dass eine Dispergierrichtung von ionischer Flüssigkeit in organischer Phase vorliegt. Durch gezielte Einstellung der Dispergierrichtung von Phase (A; ionische Flüssigkeit) in Phase (B; organische Phase) kann eine große (schnelle) Trenngeschwindigkeit erzielt werden, mit der Konsequenz dass die Größe der im Verfahren verwendeten Phasenscheider deutlich reduziert werden kann gegenüber dem Fall der umgekehrten Dispergierrichtung - Phase (B; organische Phase) in Phase (A; ionische Flüssigkeit).

Das erfindungsgemäße Verfahren kann also unabhängig davon durchgeführt werden, welche Dispergierrichtung in den vorrausgegangen Verfahrensschritten vorliegt. Liegt beispielsweise in einem vorausgegangenen Isomerisierungsschritt eine Dispergierrichtung mit Phase (B) in Phase (A) vor, weil beispielsweise bei der Isomerisierung ein deutlicher Überschuss an ionischer Flüssigkeit eingesetzt wird, kann in einer bevorzugten Ausführungsform der vorliegenden Erfindung problemlos ein Kippen der Dispergierrichtung durchgeführt werden. Das Kippen der Dispergierrichtung wird erfindungsgemäß durchgeführt, indem ein Strom, der einen Überschuss an organischer Phase enthält, vor die Koalesziervorrichtung (KV) und insbesondere auch vor einen der Koalesziervorrichtung (KV) vorgeschalteten zusätzlichen Phasenscheider rückgeleitet wird.

Aufgrund der Einstellung der Anströmgeschwindigkeit des Stroms (S1), in dem die Dispersion (D1) enthalten ist, auf Werte von 0,05 bis 150 kg/(cm² * h) beim Einleiten in die Koalesziervorrichtung (KV), wobei die vorgenannten Werte der Einströmgeschwindigkeit des Stromes (S1) auf die mittlere Querschnittsfläche der Koalesziervorrichtung (KV) bezogen sind, kann das vorstehend beschriebene Feinsttropfenproblem durch das erfindungsgemäße Verfahren besonders effizient behoben werden. Als Koalesziervorrichtung (KV) werden erfindungsgemäß Koaleszierfilter eingesetzt, wobei das Filtermaterial der Koaleszierfilter mindestens 50 Gew.-% Acrylphenolharz enthält. Die Packungsdichte der Koalesziervorrichtung (KV) beträgt erfindungsgemäß 50 bis 500 kg/m³.

Unter einer Koalesziervorrichtung (KV) wird im Rahmen der vorliegenden Erfindung jede Vorrichtung verstanden, in der eine auf Koaleszenz basierende Trennung zweier Phasen durchgeführt werden kann. Die Koalesziervorrichtung (KV) selber kann wiederum Bestandteil einer größeren Vorrichtung/Apparatur sein. Beispielsweise eine Apparatur, in der mindestens eine Koalesziervorrichtung (KV), sowie weitere Vorrichtungen enthalten sein können, wobei sich diese weiteren Vorrichtungen ebenfalls zum Auftrennen von Flüssigkeiten eignen können, aber auf einem anderen Trennmechanismus als der Koaleszenz beruhen. Dabei kann es sich beispielsweise um herkömmliche Filter oder Vorfilter handeln.

Der physikalische Mechanismus einer jeden auf Koaleszenz basierenden Trennung ist beispielsweise in dem Artikel von T. Wines et al, (Chemical Engineering; Dezember 1997; Seiten 104 bis 109) im Zusammenhang mit Koaleszierfiltern beschrieben. Dieser Trennmechanismus ist jedoch nicht auf die Verwendung von Koaleszierfiltern beschränkt, sondern gilt genauso für die Verwendung von Gestricken oder sonstiger Materialien. Bei einer auf Koaleszenz basierenden Trennung kommt es zunächst zu einem Anhaften der in der kontinuierlichen Phase enthaltenen dispergierten Tröpfchen an der Oberfläche der Koalesziervorrichtung (KV), also dem Koalesziermaterial als solchem, wobei die Koalesziervorrichtung (KV) erfindungsgemäß ein Koaleszierfilter ist und das Filtermaterial mindestens 50 Gew.-% Acrylphenolharz enthält. Die durch die Koalesziervorrichtung eingefangenen Tröpfchen koaleszieren in Form von Tropfenwachstum auf dieser Oberfläche. Anschließend erfolgt ein Abfallen oder Absetzen der durch den Koaleszenzprozess gewachsenen Tröpfchen, wodurch die Phasentrennung erzielt wird.

Der durch die Steuerung der Anströmgeschwindigkeitsdispersion (D1) erzielte Effekt kann weiter verbessert werden, da die Koalesziervorrichtungen (KV) erfindungsgemäß eine spezielle

Packungsdichte im Bereich von 50 bis 500 kg/m³ aufweisen. Ebenso lässt sich eine weitere Verbesserung beobachten, sofern dass das erfindungsgemäße Verfahren mit nicht nur einer Koalesziervorrichtung (KV) durchgeführt wird, sondern stattdessen zwei oder noch mehr Koalesziervorrichtungen (KV) eingesetzt werden, beispielsweise in einer Parallel- oder Reihenschaltung mit entsprechenden Koalesziervorrichtungen.

Durch den erfindungsgemäßen Einsatz eines Koaleszierfilters als Koalesziervorrichtungen (KV), kann eine weitere Verbesserung des Verfahrens erzielt werden, sofern der Druckverlust über den Koaleszierfilter im Bereich von 0,001 bis 1 bar liegt und/oder ein Feststofffilter ohne Klebung eingesetzt wird.

Die erfindungsgemäß eingesetzten Koaleszierfilter als Koalesziervorrichtung (KV), deren Filtermaterial mindestens 50 Gew.-% Acrylphenolharz enthält, sind (unter den für das vorliegende Verfahren relevanten Rahmenbedingungen) im Gegensatz zu anderen Koaleszierfiltern (beispielsweise Polyphenylensulfid) stabiler und/oder erhalten über einen längeren Zeitraum ihre (Trenn-) Leistung (Koaleszierwirkung) aufrecht.

Nachfolgend wird das erfindungsgemäße Verfahren zur Abtrennung von ionischen Flüssigkeiten in Koalesziervorrichtungen näher definiert.

Die Phase (A) enthält mindestens eine ionische Flüssigkeit. Beispielsweise können in der Phase (A) Gemische aus zwei oder mehr ionischen Flüssigkeiten enthalten sein, vorzugsweise enthält die Phase (A) eine ionische Flüssigkeit. Neben der ionischen Flüssigkeit können in der Phase (A) auch weitere Komponenten enthalten sein, die mit der ionischen Flüssigkeit mischbar sind. Bei solchen Komponenten kann es sich beispielsweise um Cokatalysatoren handeln, die bei Isomerisierungsreaktionen unter Verwendung von ionischen Flüssigkeiten eingesetzt werden. Bevorzugtes Beispiel für solche Cokatalysatoren sind Halogenwasserstoffe, insbesondere Chlorwasserstoff. Darüber hinaus können in der Phase (A) auch Bestandteile oder Zerfallsprodukte der ionischen Flüssigkeiten, die beispielsweise bei der durch die ionische Flüssigkeit katalysierte Reaktion entstehen können, enthalten sein, wie Aluminiumchlorid. Vorzugsweise ist bei der Phase (A) der Anteil an ionischer Flüssigkeit größer 80 Gew.-% (bezogen auf die Summe aller Komponenten der Phase (A)).

Als ionische Flüssigkeiten eignen sich im Rahmen der vorliegenden Erfindung prinzipiell alle dem Fachmann bekannten ionischen Flüssigkeiten, sofern sie die durchzuführende Reaktion wie z.B. Isomerisierung katalysieren. Ein Überblick hinsichtlich zur Katalyse von Isomerisierungsreaktionen geeigneter ionischer Flüssigkeiten kann beispielsweise WO 2011/069929 entnommen werden. Bevorzugt ist im Rahmen der vorliegenden Erfindung eine saure ionische Flüssigkeit. Vorzugsweise ist die in der Phase (A) enthaltene ionische Flüssigkeit eine saure ionische Flüssigkeit mit der Zusammensetzung K1AlₙX₍₃ₙ₊₁₎, wobei K1 ein einwertiges Kation, X gleich Halogen und 1 < n < 2,5, ist. K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die saure ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5. Vorzugsweise enthält das zumindest teilweise alkylierte Ammoniumion einen, zwei oder drei Alkylreste mit (jeweils) 1 bis 10 Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von 1 bis 3 Kohlenstoffatomen. Das heterocyclische Kation ist vorzugsweise ein Imidazoliumion oder ein Pyridiniumion.

Besonders bevorzugt enthält die saure ionische Flüssigkeit als Kation ein zumindest teilweise alkyliertes Ammoniumion und als Anion ein Chloroalumination mit der Zusammensetzung AlₙCl₍₃ₙ₊₁₎ mit 1 < n < 2,5. Beispiele für solche besonders bevorzugten ionischen Flüssigkeiten sind Trimethylammoniumchloroaluminat und Triethylammoniumchloroaluminat.

Im Rahmen der vorliegenden Erfindung weist die Phase (A) eine höhere Viskosität als die Phase (B) auf. Vorzugsweise weist die Phase (A) eine um mindestens 0,1 mPas, insbesondere um mindestens 20 mPas, höhere Viskosität aufweist als die Phase (B).

Die Phase (B) ist im Rahmen der vorliegenden Erfindung zunächst dadurch charakterisiert, dass sie gegenüber der Phase (A) eine niedrigere Viskosität aufweist. Beispielsweise kann es sich bei der Phase (B) um eine organische Phase handeln. Vorzugsweise enthält die Phase (B) mindestens einen Kohlenwasserstoff. Mehr bevorzugt enthält die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan. Besonders bevorzugt enthält die Phase (B) ein Gemisch aus Cyclohexan, MCP und mindestens einem weiteren Kohlenwasserstoff.

Im Rahmen der vorliegenden Erfindung erfolgt in Schritt a) die Bereitstellung eines Stroms (S1) enthaltend eine Dispersion (D1), in der die Phase (A) in der Phase (B) dispergiert ist. Die Dispergierrichtung (d.h. die Information, welche Phase in disperser Form in der der jeweils anderen Phase vorliegt) kann bestimmt werden, indem eine Probe, ggf. nach Zusatz eines selektiv die eine Phase einfärbenden Farbstoffs, unter einem Lichtmikroskop mit Durchlicht untersucht wird.

Die Dispersion (D1) kann nach dem Fachmann bekannten Methoden hergestellt werden, beispielsweise kann eine solche Dispersion durch intensives Verrühren der in den jeweiligen Phasen enthaltenen Komponenten erzeugt werden. Ein solcher Vorgang kann beispielsweise im Rahmen eines Isomerisierungsverfahrens von Kohlenwasserstoff unter Verwendung einer ionischen Flüssigkeit stattfinden. Die Dispersion (D1) wird (wie nachfolgend noch näher ausgeführt) bevorzugt als Oberphase aus einer Phasentrennvorrichtung entnommen, die besonders bevorzugt einer Vorrichtung nachgeschaltet ist, in der eine durch die ionische Flüssigkeit katalysierte Reaktion durchgeführt wird und in der die ionische Flüssigkeit und die organische Phase unter Rührung in Kontakt gebracht werden. In der Dispersion (D1) können die Phasen (A) und (B) in beliebigen Verhältnissen zueinander vorliegen, unter der Voraussetzung, dass die Phase (A) in der Phase (B) dispergiert ist. Vorzugsweise ist im Strom (S1) in der Dispersion (D1) die Phase (A) zu maximal 10 Gew.-%, insbesondere zu maximal 5 Gew.-% enthalten (jeweils bezogen auf die Menge an Phase (B)).

Gemäß dem erfindungsgemäßen Schritt b) erfolgt das Einleiten des Stroms (S1) in eine Koalesziervorrichtung (KV), wobei die Anströmgeschwindigkeit des Stroms (S1) 0,05 bis 150 kg/(cm² * h) bezogen auf die mittlere Querschnittsfläche der Koalesziervorrichtung (KV) beträgt und wobei die Packungsdichte der Koalesziervorrichtung (KV) 50 bis 500 kg/m³ beträgt. Die Koalesziervorrichtung (KV) ist ein Koaleszierfilter und das Filtermaterial des Koaleszierfilters enthält mindestens 50 Gew.-% Acrylphenolharz.

Die Koalesziervorrichtungen (KV) als solche sind dem Fachmann bekannt. Im Rahmen der vorliegenden Erfindung können prinzipiell alle Koalesziervorrichtungen (KV) eingesetzt werden, die dazu geeignet sind, aus einer Dispersion (D1), in der die Phase (A) in der Phase (B) dispergiert ist, die Phase (A) von der Phase (B) abzutrennen, unter der Voraussetzung, dass die Packungsdichte der Koalesziervorrichtung (KV) 50 bis 500 kg/m³ beträgt, die Koalesziervorrichtung (KV) ein Koaleszierfilter ist und das Filtermaterial des Koaleszierfilters mindestens 50 Gew.-% Acrylphenolharz enthält. Vorzugsweise erfolgt die Abtrennung vollständig oder zumindest zu großen Teilen, also mindestens 70 Gew.-%, bevorzugt 90 Gew.-%, insbesondere mindestens 99 Gew.-%, der in der Dispersion (D1) enthaltenen Phase (A) werden erfindungsgemäß unter Verwendung der Koalesziervorrichtung (KV) abgetrennt.

Weiterhin können auch zwei oder mehrere Koalesziervorrichtungen (KV) im erfindungsgemäßen Verfahren eingesetzt werden.

Koaleszierfilter können erfindungsgemäß aus allen dem Fachmann bekannten Filtermaterialien hergestellt sein, die sich zur Durchführung einer auf Koaleszens basierenden Trennung eignen, wobei das Filtermaterial der Koaleszierfilter erfindungsgemäß mindestens 50 Gew.-% Acrylphenolharz enthält. Solche Koaleszierfilter sind häufig als Kerze ausgestaltet (sogenannte Koaleszierkerzen) und können gegebenenfalls in eine größere Einheit, beispielsweise in einem Filterbehälter, integriert sein.

Im Rahmen der vorliegenden Erfindung eingesetzte Koaleszierfilter sind solche, die aus Acrylphenolharz hergestellt sind.

Solche Koaleszierfilter sind dem Fachmann bekannt, sie sind beispielsweise kommerziell erhältlich von der Firma Fuhr GmbH (Deutschland) oder von dem Hersteller CUNO Fluid Purification. Solche geeigneten Koaleszierfilter haben Feinheiten von 1-150 µm, bevorzugt 10, 25 oder 50 µm, besonders bevorzugt 10 µm. Weiterhin sind zwei Versionen bezüglich der Oberfläche möglich: gerillt und ungerillt, bevorzugt ist ungerillt. Die Kerzen des Koaleszierfilter als solche haben beispielsweise einen Innendurchmesser von 27 mm und einen Außendurchmesser von 65 mm und sind in Längen von 4" bis 60" erhältlich. Die Kerze ist vorzugsweise eine asymmetrische, harzgebundene und stützkernlose Filterkerze. Vorzugsweise enthält sie im Wesentlichen Acrylfasern, die mit Phenolharz gebunden werden.

Wie vorstehend bereits erwähnt, kann der Koaleszierfilter in eine größere Einheit, beispielsweise einen Filterbehälter, integriert sein. Vorzugsweise wird im Rahmen der vorliegenden Erfindung unter einem Koaleszierfilter, der aus Acrylphenolharz hergestellt ist, das Filtermaterial als solches verstanden. Die sonstigen Komponenten der Filtereinheit, beispielsweise der Behälter der Einheit (Filterbehälter) oder das Filtermodul, in dem das Filtermaterial eingebracht ist, können aus anderen Materialien als Acrylphenolharz hergestellt sein.

Der Begriff "hergestellt aus" bedeutet im Rahmen der vorliegenden Erfindung, dass das zur Herstellung des Filtermaterials verwendete Material Acrylphenolharz enthält. Erfindungsgemäß enthält das Filtermaterial mindestens 50 Gew.-%, bevorzugt mindestens 75 Gew.-% und insbesondere mindestens 95 Gew.-% Acrylphenolharz.

Vorzugsweise ist in Schritt b) die Anströmgeschwindigkeit des Stroms (S1) 0,5 bis 20 kg/(cm² * h), insbesondere 0,5 bis 5 kg/(cm² * h), bezogen auf die mittlere Querschnittsfläche der Koalesziervorrichtung (KV), beziehungsweise bezogen (inklusive der Bereichsangabe 0,05 bis 150 kg/(cm² * h)) auf die Querschnittsfläche des Koaleszierfilters.

Weiterhin ist erfindungsgemäß, dass die Packungsdichte der Koalesziervorrichtung (KV) 50 bis 500 kg/m³ beträgt und/oder dass mindestens zwei Koalesziervorrichtungen (KV) parallel geschaltet werden, wobei die mindestens zwei Koalesziervorrichtungen (KV) simultan und/oder alternierend betrieben werden können.

Der im erfindungsgemäßen Verfahren als Koalesziervorrichtung (KV) eingesetzte Koaleszierfilter wird bevorzugt ohne Klebung eingesetzt und/oder der Druckverlust über den Koaleszierfilter beträgt bevorzugt 0,001 bis 1 bar, mehr bevorzugt 0,001 bis 0,5 bar, besonders bevorzugt 0,001 bis 0,2 bar.

In Schritt c) erfolgt das Abtrennen der dispergierten Phase (A) von der Phase (B) in der Koalesziervorrichtung (KV). Die Durchführung des Abtrennens als solche - wobei aufgrund der Koaleszierwirkung der Koalesziervorrichtung (KV) voneinander abgetrennte Phasen (A) und (B) erhalten werden - mittels eines Koaleszierfilters als Koalesziervorrichtung (KV) ist dem Fachmann bekannt.

Gemäß Schritt d) erfolgt im erfindungsgemäßen Verfahren das Ausleiten eines Stroms (S2) umfassend mindestens 70 Gew.-%, bevorzugt mindestens 90 Gew.-% an Phase (A) aus der Koalesziervorrichtung (KV). Besonders bevorzugt ist im Strom (S2) keine oder nur eine geringe Menge an Phase (B) enthalten (< 1 Gew.-%). Die vorstehenden Angaben in Gew.-% beziehen sich auf die entsprechenden Mengenangaben, die im Strom (S1) enthalten sind.

In Schritt e) erfolgt das Ausleiten eines Stroms (S3) umfassend mindestens 70 Gew.-%, bevorzugt mindestens 90 Gew-% an Phase (B) aus der Koalesziervorrichtung (KV). Besonders bevorzugt ist im Strom (S2) keine oder nur eine geringe Menge an Phase (A) enthalten (< 1 Gew.-%). Die vorstehenden Angaben in Gew.-% beziehen sich auf die entsprechenden Mengenangaben, die im Strom (S1) enthalten sind.

Vorzugsweise wird der in Schritt a) bereitgestellte Strom (S1) aus einer der Koalesziervorrichtung (KV) vorgeschalteten Phasentrenneinheit erhalten. Bei dieser Phasentrenneinheit handelt es sich vorzugsweise um einen Phasenscheider.

Erfindungsgemäß wird dabei als Koalesziervorrichtung (KV) ein Koaleszierfilter eingesetzt.

Weiterhin ist es bevorzugt, dass der Phasentrenneinheit (wiederum) ein Reaktionsapparat oder eine Kaskade von Reaktionsapparaten vorgeschaltet ist. Bei diesem Reaktionsapparat oder der Kaskade von Reaktionsapparaten handelt es sich vorzugsweise um Vorrichtungen, die dazu geeignet sind, eine Isomerisierung von Kohlenwasserstoffen in Gegenwart von mindestens einer ionischen Flüssigkeit als Katalysator durchzuführen.

In einer bevorzugten Ausführungsform der vorliegenden Erfindung werden zusätzlich zu den vorstehend beschriebenen Schritten a) bis e) die nachfolgenden zusätzlichen Schritte f) bis k) durchgeführt, die wie folgt definiert sind:
f) Ausleitung eines Stroms (S4) aus dem Reaktionsapparat oder der Kaskade von Reaktionsapparaten, wobei (S4) eine Dispersion (D2) enthält, in der die Phase (B) in der Phase (A) dispergiert ist,
g) Einleiten eines Stroms (S5), enthaltend zu mindestens 70 Gew.-%, bevorzugt zu mindestens 90 Gew.-%, die Phase (B), in den Strom (S4), wobei der Strom (S5) aus Schritt k) rückgeführt wird, vorzugsweise werden die Ströme (S4) und (S5) mittels eines Rückorgans oder eines statischen Mischers vermischt,
h) unter Ausbildung eines Stroms (S6), enthaltend eine Dispersion (D1), in der die Phase (A) in der Phase (B) dispergiert ist,
i) Einleiten des Stroms (S6) in die Phasentrenneinheit, die der Koalesziervorrichtung (KV) vorgeschaltet ist,
j) Auftrennen des Stroms (S6) in der Phasentrenneinheit in einen Strom (S1), gemäß Schritt a) und in einen Strom (S7) enthaltend zu mindestens 70 Gew.-%, bevorzugt mindestens 90 Gew.-%, die Phase (A),
k) Abtrennung einer Teilmenge des Stroms (S1) und/oder einer Teilmenge des Stroms (S3) gemäß Schritt e) als Strom (S5) und Rückführung des Stroms (S5) nach Schritt g).

Im Rahmen der vorliegenden Erfindung kann der Strom (S5), der in Schritt g) in den Strom (S4) eingeführt wird, aus einer Teilmenge des Stromes (S1) gebildet werden. Alternativ dazu kann der Strom (S5) auch aus einer Teilmenge des Stromes (S3) gebildet werden. Gegebenenfalls kann der Strom (S5) auch aus unterschiedlichen oder gleichen Teilmengen der Ströme (S1) und (S3) gebildet werden. Vorzugsweise wird der Strom (S5) aus einer Teilmenge des Stromes (S1) gebildet. Beispielsweise werden zwischen 50 und 90 % der Ströme (S1) und/oder (S3) als Strom (S5) abgetrennt und in den Strom (S4) rückgeführt. Allerdings ist es auch denkbar, dass zumindest temporär größere Mengen oder die entsprechenden Ströme sogar vollständig rückgeleitet werden. Durch die Rückleitung von Teilmengen der Ströme (S1) und/oder (S3) als Strom (S5) und der damit verbundenen Einleitung des Stromes (S5) in den Strom (S4) wird ein Kippen der Dispergierrichtung im Strom (S4) erzielt. Kippen der Dispergierrichtung bedeutet, dass im Strom (S4) eine Dispersion (D2) enthalten ist, bei der die Phase (B) in der Phase (A) dispergiert ist und dass durch geeignete Wahl der Menge des Stromes (S5) in Schritt g) ein Strom (S6) ausgebildet wird, der die Dispersion (D1) enthält, in der die Phase (A) in der Phase (B) dispergiert ist. Sofern vor dem Koaleszierfilter (K) eine Phasentrenneinheit, insbesondere ein Phasenscheider vorgeschaltet ist, wird in der Dispersion (D1) der Anteil an Phase (A) weiter reduziert, was sich vorteilhaft auf die Trennleistung im Koaleszierfilter (K) auswirkt.

Vorzugsweise erfolgt in Schritt g) das Einleiten des Stroms (S5) in den Strom (S4) in einer Rühr- oder Mischvorrichtung, in der der Strom (S6) gemäß Schritt h) ausgebildet wird.

Weiterhin ist es bevorzugt, dass das Phasenverhältnis der Phase (A) zur Phase (B) in der im Strom (S6) enthaltenen Dispersion (D1) ≤ 3 [kg/kg], bevorzugt ≤ 0,9 [kg/kg], ist.

Weiterhin ist es bevorzugt, dass der Strom (S4) aus einer Isomerisierung in Gegenwart einer ionischen Flüssigkeit, insbesondere einer Isomerisierung von Methylcyclopentan (MCP) zu Cyclohexan in Gegenwart einer ionischen Flüssigkeit, erhalten wird.

Weiterhin ist es bevorzugt, dass in Schritt k) die Abtrennung von Strom (S5) aus Strom (S1) außerhalb der Phasentrenneinheit erfolgt.

Gegebenenfalls können der aus der Phasentrenneinheit gemäß Schritt j) abgetrennte Strom (S7) und/oder der aus der Koalesziervorrichtung (KV) ausgeleitete Strom (S2) gemäß Schritt d), die jeweils die Phase (A) enthalten, in den Reaktionsapparat oder die Kaskade von Reaktionsapparaten rückgeleitet werden. Gegebenenfalls können der Strom (S7) und/oder der Strom (S2) auch an eine andere Stelle des erfindungsgemäßen Verfahrens rückgeleitet werden, beispielswiese in eine Misch-oder Rührvorrichtung, um die Konzentration der Phase (A) in der Dispersion (D1) zu steuern.

Aus dem Strom (S3) wird im Rahmen der vorliegenden Erfindung vorzugsweise Cyclohexan isoliert. Verfahren und Vorrichtungen zur Abtrennung von Cyclohexan aus dem Strom (S3), insbesondere wenn es sich um ein Kohlenwasserstoffgemisch handelt, sind dem Fachmann bekannt. Gegebenenfalls können vor der Abtrennung des Cyclohexans noch weitere Aufreinigungsschritte (beispielsweise eine Wäsche mit einer wässrigen und/oder alkalischen Phase) durchgeführt werden, die dem Fachmann bekannt sind.

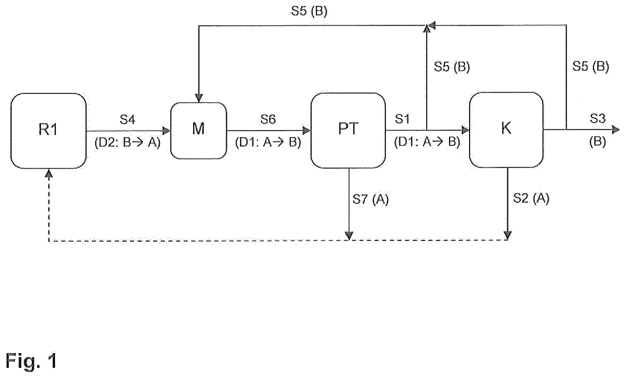

In Figur 1 wird das erfindungsgemäße Verfahren (einer Ausgestaltung) der vorstehend beschriebenen bevorzugten Ausführungsform nochmals verdeutlicht. Gemäß Figur 1 wird das Verfahren so durchgeführt, dass sowohl aus dem Strom (S1) als auch aus dem Strom (S3) eine Teilmenge als Strom (S5) in den Strom (S4) rückgeführt wird. Zum besseren Verständnis sind in Figur 1 unter den jeweiligen Strömen in Klammern die darin enthaltenen Hauptkomponenten angegeben. Bei den Strömen (S1), (S4) und (S6) ist im jeweiligen Klammerausdruck auch die Dispergierrichtung der jeweiligen Dispersionen mit berücksichtigt, wobei der Pfeil die Dispergierrichtung zum Ausdruck bringt. Dies bedeutet, dass beispielsweise die im Strom (S4) enthaltene Dispersion (D2) eine Phase (B) aufweist, die in der Phase (A) dispergiert ist. In Figur 1 erfolgt die Einleitung des Stromes (S5) in den Strom (S4) in einer Mischvorrichtung (M). Durch die gestrichelte Linie ist angedeutet, dass die Ströme (S7) und/oder (S2) gegebenenfalls auch in den Reaktionsapparat oder eine Kaskade von Reaktionsapparaten (R1) rückgeleitet werden können. PT in Figur 1 bedeutet Phasentrenneinheit und K bedeutet Koaleszierfilter.

## Patentansprüche

1. Verfahren zur Abtrennung einer Phase (A), die mindestens eine ionische Flüssigkeit enthält, von einer Phase (B), wobei die Phase (A) eine höhere Viskosität als die Phase (B) aufweist, umfassend die folgenden Schritte:
a) Bereitstellung eines Stroms (S1) enthaltend eine Dispersion (D1), in der die Phase (A) in der Phase (B) dispergiert ist,
b) Einleiten des Stroms (S1) in eine Koalesziervorrichtung (KV), wobei die Anströmgeschwindigkeit des Stroms (S1) 0,05 bis 150 kg/(cm² * h) bezogen auf die mittlere Querschnittsfläche der Koalesziervorrichtung (KV) beträgt, wobei die Packungsdichte der Koalesziervorrichtung (KV) 50 bis 500 kg/m³ beträgt, wobei die Koalesziervorrichtung (KV) ein Koaleszierfilter ist und das Filtermaterial des Koaleszierfilters mindestens 50 Gew.-% Acrylphenolharz enthält.
c) Abtrennen der dispergierten Phase (A) von der Phase (B) in der Koalesziervorrichtung (KV),
d) Ausleiten eines Stroms (S2) umfassend mindestens 70 Gew.-%, bevorzugt mindestens 90 Gew.-% an Phase (A) aus der Koalesziervorrichtung (KV) und
e) Ausleiten eines Stroms (S3) umfassend mindestens 70 Gew.-%, bevorzugt mindestens 90 Gew.-% an Phase (B) aus der Koalesziervorrichtung (KV).

2. Verfahren gemäß Anspruch 1, **dadurch gekennzeichnet, dass** die Koalesziervorrichtung (KV) ein Koaleszierfilter oder ein Gestrick, vorzugsweise ein Koaleszierfilter, ist.

3. Verfahren gemäß Anspruch 1 oder 2, **dadurch gekennzeichnet, dass**
i) die Anströmgeschwindigkeit des Stroms (S1) 0,5 bis 20 kg/(cm² * h), insbesondere 0,5 bis 5 kg/(cm² * h), bezogen auf die mittlere Querschnittsfläche der Koalesziervorrichtung (KV) beträgt, und/oder
ii) mindestens zwei Koalesziervorrichtungen (KV) parallel geschaltet werden, wobei die mindestens zwei Koalesziervorrichtungen (KV) simultan und/oder alternierend betrieben werden können.

4. Verfahren gemäß einem der Ansprüche 1 bis 3, **dadurch gekennzeichnet, dass** die Koalesziervorrichtung (KV) ein Koaleszierfilter ist und
i) der Koaleszierfilter ohne Klebung eingesetzt wird, und/oder
ii) der Druckverlust über den Koaleszierfilter 0,001 bis 1 bar, bevorzugt 0,001 bis 0,5 bar, besonders bevorzugt 0,001 bis 0,2 bar, beträgt.

5. Verfahren gemäß einem der Ansprüche 1 bis 4, **dadurch gekennzeichnet, dass** die Phase (B) mindestens einen Kohlenwasserstoff enthält, vorzugsweise enthält die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan.

6. Verfahren gemäß einem der Ansprüche 1 bis 5, **dadurch gekennzeichnet, dass** die in der Phase (A) enthaltene ionische Flüssigkeit eine saure ionische Flüssigkeit mit der Zusammensetzung K1AlₙX₍₃ₙ₊₁₎ ist, wobei K1 ein einwertiges Kation, X gleich Halogen und 1 < n < 2,5 ist, vorzugsweise enthält die saure ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5.

7. Verfahren gemäß einem der Ansprüche 1 bis 5, **dadurch gekennzeichnet, dass** im Strom (S1) in der Dispersion (D1) die Phase (A) zu maximal 5 Gew.-% vorliegt.

8. Verfahren gemäß einem der Ansprüche 1 bis 7, **dadurch gekennzeichnet, dass** der Strom (S1) aus einer der Koalesziervorrichtung (KV) vorgeschalteten Phasentrenneinheit erhalten wird, der wiederum ein Reaktionsapparat oder eine Kaskade von Reaktionsapparaten vorgeschaltet ist.

9. Verfahren gemäß Anspruch 8, **dadurch gekennzeichnet, dass** die Phasentrenneinheit ein Phasenscheider ist.

10. Verfahren gemäß Anspruch 8 oder 9 umfassend die folgenden zusätzlichen Schritte:
f) Ausleitung eines Stroms (S4) aus dem Reaktionsapparat oder der Kaskade von Reaktionsapparaten gemäß Anspruch 8, wobei (S4) eine Dispersion (D2) enthält, in der die Phase (B) in der Phase (A) dispergiert ist,
g) Einleiten eines Stroms (S5), enthaltend zu mindestens 70 Gew-.%, bevorzugt zu mindestens 90 Gew-.%, die Phase (B), in den Strom (S4), wobei der Strom (S5) aus Schritt k) rückgeführt wird,
h) unter Ausbildung eines Stroms (S6), enthaltend eine Dispersion (D1), in der die Phase (A) in der Phase (B) dispergiert ist,
i) Einleiten des Stroms (S6) in die Phasentrenneinheit, die der Koalesziervorrichtung (KV) vorgeschaltet ist,
j) Auftrennen des Stroms (S6) in der Phasentrenneinheit in einen Strom (S1), gemäß Schritt a) von Anspruch 1, und in einen Strom (S7) enthaltend zu mindestens 70 Gew.-%, bevorzugt mindestens 90 Gew.-%, die Phase (A),
k) Abtrennung einer Teilmenge des Stroms (S1) und/oder einer Teilmenge des Stroms (S3) gemäß Schritt e) von Anspruch 1 als Strom (S5) und Rückführung des Stroms (S5) nach Schritt g).

11. Verfahren gemäß Anspruch 10, **dadurch gekennzeichnet, dass** in Schritt g) das Einleiten des Stroms (S5) in den Strom (S4) in einer Rühr- oder Mischvorrichtung erfolgt, in der der Strom (S6) gemäß Schritt h) ausgebildet wird.

12. Verfahren gemäß Anspruch 10 oder 11, **dadurch gekennzeichnet, dass** das Phasenverhältnis der Phase (A) zur Phase (B) in der im Strom (S6) enthaltenen Dispersion (D1) ≤ 3 [kg/kg], bevorzugt ≤ 0,9 [kg/kg] ist.

13. Verfahren gemäß einem der Ansprüche 10 bis 12, **dadurch gekennzeichnet, dass** der Strom (S4) aus einer Isomerisierung erhalten wird, vorzugsweise einer Isomerisierung in Gegenwart einer ionischen Flüssigkeit, insbesondere einer Isomerisierung von Methylcyclopentan (MCP) zu Cyclohexan in Gegenwart einer ionischen Flüssigkeit.

14. Verfahren nach einem der Ansprüche 10 bis 13, **dadurch gekennzeichnet, dass** in Schritt k) die Abtrennung von Strom (S5) aus Strom (S1) außerhalb der Phasentrenneinheit erfolgt.

15. Verfahren gemäß einem der Ansprüche 1 bis 14, **dadurch gekennzeichnet, dass** aus dem Strom (S3) Cyclohexan isoliert wird.

## Claims

1. A process for separating a phase (A) comprising at least one ionic liquid from a phase (B), where the phase (A) has a higher viscosity than the phase (B), which comprises the following steps:
a) provision of a stream (S1) comprising a dispersion (D1) in which the phase (A) is dispersed in the phase (B),
b) introduction of the stream (S1) into a coalescing device (KV), where the inflow rate of the stream (S1) is from 0.05 to 150 kg/ (cm² * h) based on the average cross-sectional area of the coalescing device (KV), where the packing density of the coalescing device (KV) is from 50 to 500 kg/m³, where the coalescing device (KV) is a coalescing filter and the filter material of the coalescing filter comprises at least 50% by weight of acrylphenol resin,
c) separation of the disperse phase (A) from the phase (B) in the coalescing device (KV),
d) discharge of a stream (S2) comprising at least 70% by weight, preferably at least 90% by weight, of phase (A) from the coalescing device (KV) and
e) discharge of a stream (S3) comprising at least 70% by weight, preferably at least 90% by weight, of phase (B) from the coalescing device (KV).

2. The process according to claim 1, wherein the coalescing device (KV) is a coalescing filter or a knitted fabric, preferably a coalescing filter.

3. The process according to claim 1 or 2, wherein
i) the inflow rate of the stream (S1) is from 0.5 to 20 kg/(cm² * h), in particular from 0.5 to 5 kg/(cm² * h), based on the average cross-sectional area of the coalescing device (KV), and/or
ii) at least two coalescing devices (KV) are connected in parallel, with the at least two coalescing devices (KV) being operatable simultaneously and/or alternately.

4. The process according to any of claims 1 to 3, wherein the coalescing device (KV) is a coalescing filter and
i) the coalescing filter is used without adhesive bonding and/or
ii) the pressure drop over the coalescing filter is from 0.001 to 1 bar, preferably from 0.001 to 0.5 bar, particularly preferably from 0.001 to 0.2 bar.

5. The process according to any of claims 1 to 4, wherein the phase (B) comprises at least one hydrocarbon, with the phase (B) preferably comprising, as hydrocarbon, cyclohexane or a mixture of cyclohexane with at least one further hydrocarbon selected from among methylcyclopentane (MCP), n-hexane, isohexane, n-heptane, isoheptane and dimethylcyclopentane.

6. The process according to any of claims 1 to 5, wherein the ionic liquid comprised in the phase (A) is an acidic ionic liquid having the composition K1AlₙX₍₃ₙ₊₁₎, where K1 is a monovalent cation, X is halogen and 1 < n < 2.5, with the acidic ionic liquid preferably comprising an at least partially alkylated ammonium ion or a heterocyclic cation as cation and/or a chloroaluminate ion having the composition AlₙCl₍₃ₙ₊₁₎ where 1 < n < 2.5 as anion.

7. The process according to any of claims 1 to 5, wherein the dispersion (D1) in the stream (S1) preferably comprises a maximum of 5% by weight of the phase (A).

8. The process according to any of claims 1 to 7, wherein the stream (S1) is obtained from a phase separation unit which is located upstream of the coalescing device (KV) and is in turn located upstream of a reaction apparatus or a cascade of reaction apparatuses.

9. The process according to claim 8, wherein the phase separation unit is a phase separator.

10. The process according to claim 8 or 9, which comprises the following additional steps:
f) discharge of a stream (S4) from the reaction apparatus or the cascade of reaction apparatuses according to claim 8, where (S4) comprises a dispersion (D2) in which the phase (B) is dispersed in the phase (A),
g) introduction of a stream (S5) comprising at least 70% by weight, preferably at least 90% by weight, of the phase (B) into the stream (S4), where the stream (S5) is recirculated from step k),
h) to form a stream (S6) comprising a dispersion (D1) in which the phase (A) is dispersed in the phase (B),
i) introduction of the stream (S6) into the phase separation unit located upstream of the coalescing device (KV),
j) separation of the stream (S6) in the phase separation unit into a stream (S1) as per step a) of claim 1 and a stream (S7) comprising at least 70% by weight, preferably at least 90% by weight, of the phase (A),
k) separating-off of part of the stream (S1) and/or part of the stream (S3) as per step e) of claim 1 as stream (S5) and recirculation of the stream (S5) to step g).

11. The process according to claim 10, wherein the introduction of the stream (S5) into the stream (S4) in step g) is effected in a stirring or mixing apparatus in which the stream (S6) as per step h) is formed.

12. The process according to claim 10 or 11, wherein the phase ratio of the phase (A) to the phase (B) in the dispersion (D1) comprised in the stream (S6) is ≤ 3 [kg/kg], preferably ≤ 0.9 [kg/kg].

13. The process according to any of claims 10 to 12, wherein the stream (S4) is obtained from an isomerization, preferably an isomerization in the presence of an ionic liquid, in particular an isomerization of methylcyclopentane (MCP) to cyclohexane in the presence of an ionic liquid.

14. The process according to any of claims 10 to 13, wherein the separation of stream (S5) from stream (S1) in step k) is effected outside the phase separation unit.

15. The process according to any of claims 1 to 14, wherein cyclohexane is isolated from the stream (S3) .

## Revendications

1. Procédé de séparation d'une phase (A), qui contient au moins un liquide ionique, d'une phase (B), la phase (A) présentant une viscosité plus élevée que la phase (B), comprenant les étapes suivantes :
a) la préparation d'un courant (S1) contenant une dispersion (D1), dans laquelle la phase (A) est dispersée dans la phase (B),
b) l'introduction du courant (S1) dans un dispositif de coalescence (KV), la vitesse d'écoulement du courant (S1) étant de 0,05 à 150 kg/(cm²*h), par rapport à la surface de section transversale moyenne du dispositif de coalescence (KV), la densité de remplissage du dispositif de coalescence (KV) étant de 50 à 500 kg/m³, le dispositif de coalescence (KV) étant un filtre à coalescence et le matériau filtrant du filtre à coalescence contenant au moins 50 % en poids d'une résine d'acrylphénol,
c) la séparation de la phase dispersée (A) de la phase (B) dans le dispositif de coalescence (KV),
d) le déchargement d'un courant (S2) comprenant au moins 70 % en poids, de préférence au moins 90 % en poids, de phase (A), du dispositif de coalescence (KV) et
e) le déchargement d'un courant (S3) comprenant au moins 70 % en poids, de préférence au moins 90 % en poids, de phase (B), du dispositif de coalescence (KV).

2. Procédé selon la revendication 1, **caractérisé en ce que** le dispositif de coalescence (KV) est un filtre à coalescence ou un tricot, de préférence un filtre à coalescence.

3. Procédé selon la revendication 1 ou 2, **caractérisé en ce que**
i) la vitesse d'écoulement du courant (S1) est de 0,5 à 20 kg/(cm²*h), notamment de 0,5 à 5 kg/(cm²*h), par rapport à la surface de section transversale moyenne du dispositif de coalescence (KV), et/ou
ii) au moins deux dispositifs de coalescence (KV) sont raccordés en parallèle, lesdits au moins deux dispositifs de coalescence (KV) pouvant être exploités simultanément et/ou en alternance.

4. Procédé selon l'une quelconque des revendications 1 à 3, **caractérisé en ce que** le dispositif de coalescence (KV) est un filtre à coalescence et
i) le filtre à coalescence est utilisé sans collage et/ou
ii) la perte de charge dans le filtre à coalescence est de 0,001 à 1 bar, de préférence de 0,001 à 0,5 bar, de manière particulièrement préférée de 0,001 à 0,2 bar.

5. Procédé selon l'une quelconque des revendications 1 à 4, **caractérisé en ce que** la phase (B) contient au moins un hydrocarbure, la phase (B) contenant de préférence en tant qu'hydrocarbure du cyclohexane ou un mélange de cyclohexane avec au moins un autre hydrocarbure, choisi parmi le méthylcyclopentane (MCP), le n-hexane, l'iso-hexane, le n-heptane, l'iso-heptane ou le diméthylcyclopentane.

6. Procédé selon l'une quelconque des revendications 1 à 5, **caractérisé en ce que** le liquide ionique contenu dans la phase (A) est un liquide ionique acide de composition K1AlₙX₍₃ₙ₊₁₎, K1 étant un cation monovalent, X étant un halogène et 1 < n < 2,5, le liquide ionique acide contenant de préférence en tant que cation un ion ammonium au moins partiellement alkylé ou un cation hétérocyclique et/ou en tant qu'anion un ion chloroaluminate de composition AlₙCl₍₃ₙ₊₁₎ avec 1 < n < 2,5.

7. Procédé selon l'une quelconque des revendications 1 à 5, **caractérisé en ce que** la phase (A) est présente à hauteur d'au plus 5 % en poids dans la dispersion (D1) dans le courant (S1).

8. Procédé selon l'une quelconque des revendications 1 à 7, **caractérisé en ce que** le courant (S1) est obtenu à partir d'une unité de séparation de phases raccordée en amont du dispositif de coalescence (KV), en amont de laquelle un appareil de réaction ou une cascade d'appareils de réaction est raccordé.

9. Procédé selon la revendication 8, **caractérisé en ce que** l'unité de séparation de phases est un séparateur de phases.

10. Procédé selon la revendication 8 ou 9, comprenant les étapes supplémentaires suivantes :
f) le déchargement d'un courant (S4) de l'appareil de réaction ou de la cascade d'appareils de réaction selon la revendication 8, (S4) contenant une dispersion (D2) dans laquelle la phase (B) est dispersée dans la phase (A),
g) l'introduction d'un courant (S5), contenant au moins 70 % en poids, de préférence au moins 90 % en poids, de la phase (B), dans le courant (S4), le courant (S5) étant recyclé depuis l'étape k),
h) pour former un courant (S6), contenant une dispersion (D1), dans laquelle la phase (A) est dispersée dans la phase (B),
i) l'introduction du courant (S6) dans l'unité de séparation de phases, qui est raccordée en amont du dispositif de coalescence (KV),
j) la séparation du courant (S6) dans l'unité de séparation de phases en un courant (S1) selon l'étape a) de la revendication 1, et en un courant (S7) contenant au moins 70 % en poids, de préférence au moins 90 % en poids, de la phase (A),
k) la séparation d'une partie du courant (S1) et/ou d'une partie du courant (S3) selon l'étape e) de la revendication 1 en tant que courant (S5) et le recyclage du courant (D5) selon l'étape g).

11. Procédé selon la revendication 10, **caractérisé en ce qu'**à l'étape g), l'introduction du courant (S5) dans le courant (S4) a lieu dans un dispositif d'agitation ou de mélange, dans lequel le courant (S6) est formé selon l'étape h).

12. Procédé selon la revendication 10 ou 11, **caractérisé en ce que** le rapport de phases entre la phase (A) et la phase (B) dans la dispersion (D1) contenue dans le courant (S6) est ≤ 3 [kg/kg], de préférence ≤ 0,9 [kg/kg].

13. Procédé selon l'une quelconque des revendications 10 à 12, **caractérisé en ce que** le courant (S4) est obtenu par une isomérisation, de préférence une isomérisation en présence d'un liquide inique, notamment une isomérisation de méthylcyclopentane (MCP) en cyclohexane en présence d'un liquide ionique.

14. Procédé selon l'une quelconque des revendications 10 à 13, **caractérisé en ce qu'**à l'étape k), la séparation du courant (S5) du courant (S1) a lieu à l'extérieur de l'unité de séparation de phases.

15. Procédé selon l'une quelconque des revendications 1 à 14, **caractérisé en ce que** du cyclohexane est isolé à partir du courant (S3).
